# EUROPEAN PATENT APPLICATION

(11) **EP 2 052 698 A1**
(43) Date of publication of application: **29.04.2009**
(21) Application number: 06807913.6
(22) Date of filing: 08.08.2006
(51) Int. Cl.: A61F 2/14

(54) **IMPLANTABLE OPTICAL SYSTEM, METHOD FOR DEVELOPING IT AND APPLICATIONS**

(71) Applicant: Fundacion Inasmet, 20009, Guipuzcoa (San Sebastian) (ES)
(72) Inventor: GARAGORRI GANCHEGUI, Nerea, 20009 Guipuzcoa (San Sebastian) (ES); MADARIETA PARDO, Iratxe, 20009 Guipuzcoa (San Sebastian) (ES); OLALDE GRAELLS, Beatriz, 20009 Guipuzcoa (San Sebastian) (ES)
(74) Representative: Carpintero Lopez, Francisco
(86) International application number: PCT/ES2006/000467
(87) International publication number: WO 2008/020087

(57) **Abstract**

The present invention refers to an implantable optical system comprising a central optical part and an annular anchoring part, where said annular part comprises animals cells, including human cells, that encourage the integration of the implant into the ocular tissue of the patient, as well as a system for dosing chemical compounds directed at a particular function, creating a stabilising microenvironment for the presence of the implant in the tissue. A method for obtaining said system by polymerisation and its applications in various types of ocular disorders is also described.

## Description

### FIELD OF THE INVENTION

The field of the present invention is that of Ophthalmology. More specifically, this invention refers to an implantable optical system applied to corneal disorders. It comprises one central optical part and one annular peripheral part that comprises animal cells, which encourages the integration of the implant into the corneal tissue of the patient. The invention also describes the method for obtaining the system and its applications in ocular disorders.

### BACKGROUND

Disorders affecting the cornea constitute one of the main causes of blindness in the world, only surpassed in significance by cataracts. Epidemiology of corneal blindness is complicated and is accompanied by a variety of infectious and inflammatory ocular diseases that cause corneal scars finally leading to blindness. Also, the prevalence of corneal diseases varies between countries and even populations (1). Approximately 10 million people suffer corneal blindness in the world (1, 3) either due to genetic or acquired conditions.

The most successful technique for treating corneal problems is corneal transplant, where success is mainly dependent on the type of patient or recipient. Corneal transplant is successful in approximately 90% of patients considered of "low risk" in developed countries (1). These are characterised by suffering loss of vision due to corneal scars caused by trauma, keratoconus or endothelial failures (due to dystrophy or previous operations). In spite of the high percentage success rate for corneal transplant in these patients, there are important limitations with currently used techniques. These include faults in grafts due to immunological rejection or endothelial dysfunction, significant astigmatism due to topological irregularity, unpredictability in refractive error and other less common although problematic issues, such as infections in sutures, recurrent corneal diseases, etc.

The probability of corneal transplant survival diminishes significantly in patients considered of "high risk" who suffer corneal inflammatory diseases (herpes simplex or zoster), dry eye (Sjögren's syndrome), severe and generalised superficial ocular diseases (Stevens-Johnson syndrome, ocular cicatricial pemphigoid, chemical/thermal burns, etc.) and some congenital abnormalities (e.g. Peter's anomaly) where the percentage success rate is close to zero. The same fate befalls patients who have previously suffered immunological rejection. Corneal blindness due to infection (especially trachoma) is endemic in many parts of the world; the situation is aggravated in developing countries as they lack eye banks (1, 2), post-operative medicines and the social context required for routine post-operative monitoring.

It is estimated that approximately 40,000 corneal transplants per year are currently carried out in the United States. In Spain, the figure is approximately 1,500 increasing by 10% per year. These figures could vary as some trends have recently been emerging for reductions in donations of corneal tissues due to 1) increase in the number of serological tests required by the eye bank systems and 2) the increase in the number of people requesting refractive surgery (4, 5, 6).

Corneal blindness is considered particularly tragic and frustrating because the majority of patients have intact retinas and optic nerves. The ideal situation would be to obtain artificial corneas to completely replace those originating from human donors. So there has been a hope for some time that some form or artificial cornea or keratoprosthesis could be developed that would meet these patient's needs.

The history of keratoprosthesis started in the 18th century (7, 8). The modern era of such development started in 1950 with the use of various forms of methyl methacrylate (PMMA). The most significant of these were the Dohlman, Strampelli and Cardona prostheses, comprising a central optical rigid part of PMMA that perforates the cornea, which is surrounded by various tissues to achieve anchoring of the prosthesis (9, 10, 11, 12, 13). The common challenged faced by these implants has been the maintenance of optical clarity, biointegration in the receptor tissue and the prevention of extrusion. The anchoring systems of the Dohlman and Cardona prostheses combine different procedures and tissues (e.g. fascia, sclera, periosteum, etc.) to minimise extrusion. These attempts bring with them serious complications including extrusion, endophthalmitis, glaucoma and retinal detachment (14, 15, 16).

One of the latest trends in these types of implant is the incorporation of porous materials to improve the integration between the core (lens or central part) and the skirt (ring surrounding the core or annular part) and between the skirt and the receptor tissue. These materials include Teflon, Gore-Tex (42, 43, 44), Dacron, carbon fibre and rubber (7, 17, 18). Related procedures have used soft materials and/or modifications of their surfaces with the aim of increasing biointegration (19, 20, 21, 22, 23).

Chirila's group, leader in research into keratoprosthesis over the last decade, has developed a new implant (Chirila KPro) based on the use of a hydrophilic polymer, 2-hydroxyethyl methacrylate or PHEMA, which is used for a porous skirt (PHEMA sponge) and a transparent core (PHEMA gel). Both components "core and skirt" are bound by a polymeric mesh that penetrates both parts, preventing cracks, filtrations and the growth of tissue at this interface (8, 3, 24, 25, 26, 27). But there have been post-operative complications in experimental animals and the majority of them were caused by the lack of mechanical strength of the material resulting in breakages of the skirt at the surgical sutures. Unfortunately, this weakness is inherent in PHEMA sponges. Subsequently, a KPro type II has been developed with the same characteristics but finer than the previous model with the aim of reducing the dependence between the conjunctiva lid and mechanical resistance of the sponge at the sutures. It was implanted in 10 patients and although the results were much more satisfactory than those with the KPro type 1 prosthesis, recurrent inflammations were observed in the cornea. The KPro type II prosthesis has given rise to a new generation of keratoprosthesis known as AlphaCorTM (65, 66) recently approved by the American Food and Drug Administration agency (Reg. No. K013756) (28).

However, in spite of the design improvements in the devices and the materials used, complications related to the maintenance of optical clarity, epithelial covering, poor tissue integration and extrusion have prevented current keratoprosthesis from being clinically effective (3, 7, 15, 29, 30, 31).

Another alternative is in the development of corneal layers *in vitro.* In 1999, Griffith and her team reconstructed the cornea using biological techniques to recreate the epithelium, stroma and endothelium layer (36, 33). They developed a multi-layer cornea based on immortalised cells that morphologically maintained the original organisation of the tissue (37).

Also in the same year, Germain and her team developed an epithelium, cultivating epithelial cells on collagen gels (32).

They showed epithelium with a thickness of 4-5 cell layers in addition to a basal membrane but they did not achieve the reconstruction of complete corneas.

Recently, Marmo and Back have proposed the use of epithelial cells or limbic stem cells on the body of a lens to activate corneal epithelialisation. They also propose the use of epithelial portions as an active covering (72).

Biological techniques and tissue engineering enable us to use cells as authors in the reconstruction of tissues. These cells, isolated from their tissue and in the presence of a support (synthetic, natural or mixed in origin) are capable of generating an extracellular matrix that is morphologically similar to the original and composed of collagens and other adhesive fibres (45).

Hydrogels are good candidates as cellular supports. They are hydrophilic polymers that form three dimensional networks, which have the capability to capture much water without dissolving in it. They also exhibit good biocompatibility and high permeability to oxygen and other nutrients so they are the materials of choice in cell therapy (38), tissue regeneration (39) and controlled release of active substances (65).

These advantages have led to the use of photopolymerisable hydrogels as encapsulating materials, cellular supports and coverings in applications for prevention (prevention of thrombosis 58, 59), diagnostics (biosensor covering 60) and therapeutics: encapsulation of cells from the cartilage (45), bone (46), osseous medulla (47, 48, 49, 50), embryonic tissue (51), pancreas (52), cardiac valves (53), etc., encapsulation of DNA (54), nucleotides (55), growth factors (56) and other proteins (57), etc.

The photopolymerisation technique enables the encapsulation not only of cells and cell components but also of other components in these photopolymerisable materials (64). It enables the encapsulation of particles that act as active substance release systems, such as the micro and/or nano spheres that are composed of biodegradable polymers, allowing controlled release of pharmaceuticals specifically within the desired therapeutic range. Hubbell and co-workers proposed the technique of photopolymerisation to make controlled release materials and systems (65).

Controlled release systems for active substances have been used since the beginning of the 20th century (61) and were originated in the need to improve the administration of pharmaceuticals. These are systems that can control the dose released and systems capable of directing these active molecules specifically to the target organs.

These systems have been investigated for application in various therapeutic interventions in the eye. The main demands are for biodegradable implants for treating disorders in the retina, vitreous or glaucoma with a wide range of therapeutic agents (66-71).

Among biodegradable materials, the most common has been the use of poly(DL-lactic acid) and/or poly(D-lactic-co-glycolic acid) due to the total absence of toxicity of the degradation products and their controllable degradation speed (62).

The release of conventional active principles from poly(lactic acid) /poly(glycolic acid) micro spheres generally occurs by diffusion through the polymer matrix as well as through the pores in the polymeric structure. However, biodegradation of the polymer matrix and dissolution of the degraded polymer continually changes the microspheres' geometry and the texture of the polymer matrix. As a result, the model for the release of active agents is a combination of diffusion and degradation (63).

In view of the requirements and limitations of the state of the art, the authors of the present invention have developed a new model of keratoprosthesis making simultaneous use of hydrogels and biological techniques and advancing the concept of stromal implant. This provides biological intelligence to traditional keratoprosthesis and makes it susceptible to modification, depending on the conditions of the receptor tissue and the specific requirements of each patient, so that the same keratoprosthesis performs a double function, optical and therapeutic.

This keratoprosthesis comprises a central optical part and an annular part (core and skirt). The main novelty resides in that the annular part, skirt, includes cellular components, able to secrete collagen fibres and proteoglycans to improve the integration of the implant and the maintenance of corneal transparency, and a system for dosing chemical compounds directed at a particular function (e.g. anti-inflammatory, regenerative substances, etc.) creating a stabilising microenvironment for the presence of the implant in the tissue.

The design of this bicompartmental lens can be adapted to the requirements of the patient and will depend on the pathological state of the stroma and corneal epithelium. Due to its characteristics, the implant can also be used in the development of other implant types in patients with different ocular disorders.

### BRIEF DESCRIPTION OF THE FIGURES

- Fig. 1.: General figure of the optical system shown in plan and perspective views. A: is the central optical part, B: is the annular part including cells (B1) and active spheres (B2).
- Fig. 2.: Schema of different types of implants. A) and B) Lenses implanted in the anterior stroma; C) Lens implanted in medial stroma; and D) Lens implanted in posterior stroma. (Ep): Epithelium; (Es a): Anterior stroma; (Es m): Medial stroma; (Es p): Posterior stroma; (En) Endothelium.

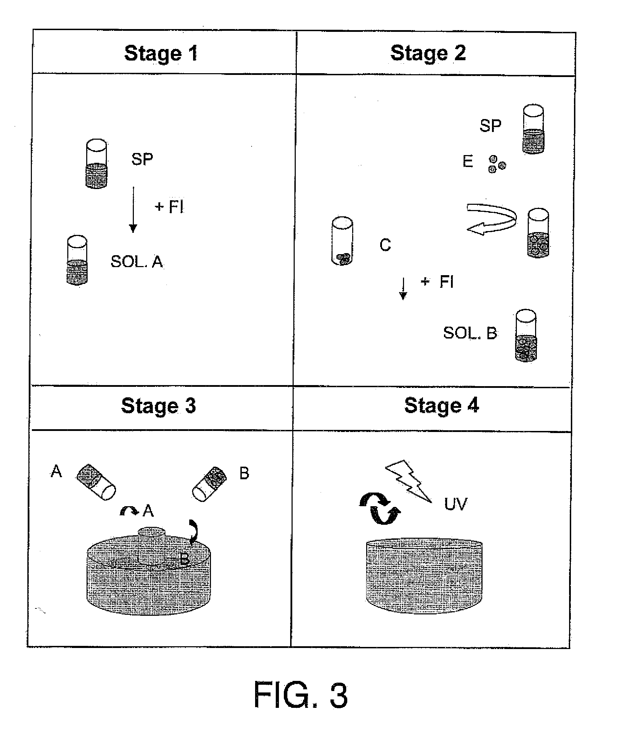

- Fig. 3.: Schema of the process for obtaining bicompartmental lenses by photopolymerisation. Stage 1) Preparation of the solution of the central optical part (SOL. A), which consists of a polymeric solution (SP) and a photoinitiator (FI); Stage 2) Preparation of the solution of the annular part or ring (SOL. B) comprising a polymeric solution (SP), photoinitiator (FI), cells (C) and active spheres (E) as the therapeutic system; Stage 3) Placing the solutions A and B from stages 1 and 2 in specific moulds; and Stage 4) Photopolymerisation of the system with UV light to obtain a bicompartmental lens.
- Fig. 4.: Schema of the photopolymerisation stage for obtaining the bicompartmental lens. 1) Filling the mould with the solutions A and B from Phases 1 and 2 (Fig. 3.) respectively; 2) Closure of the mould; 3) polymerisation and homogenisation of the sample; 4) Opening the lid and removing the separator; 5) Closure of the lid; 6) Polymerisation and homogenisation of the sample; 7) Opening the lid and extracting the bicompartmental lens.
- Fig. 5.: Three dimensional image of the PEGDA (H) hydrogel containing live primary stromal cells (CEP) taken by confocal microscopy at 63x.

### OBJECT OF THE INVENTION

The main object of the invention is to provide a new model of implantable optical system formed by a central optical part and an annular part for anchoring with live animal cells, including human.

Another purpose of the invention refers to the procedure for obtaining said implantable optical system.

The implantable optical system obtained by said procedure is also an object of the invention.

Finally, another object of the invention is the use of the implantable optical system in corneal and lens disorders, specifically in the development of corneal implants, such as intra-stromal lenses or keratoprosthesis and intra-ocular lenses.

### DESCRIPTION OF THE INVENTION

In order to cover the requirements of the current state of the art in relation to traditional keratoprosthesis, the authors of the present invention have developed a new model of implantable optical system addressing both on the design of the implant and in the nature of corneal disorders.

The present invention provides a hybrid system comprising of a mixture of synthetic materials with live materials, non-biodegradable materials with biodegradable materials, inert with chemically active substances, all brought together into an implantable system enabling each of the components to perform their function.

A main aspect of the invention is an implantable optical system that comprises a central optical part and an annular anchoring part comprising live animal cells, including human cells.

In a particular embodiment, the cells may come from the patient that will be implanted and/or from a donor. These cells, depending on the degree of ocular disorder and the objective to be repaired, can be selected from stem cells, primary keratocytes, fibroblasts, myofibroblasts, cells of various origins susceptible to differentiation into stromal cells, genetically modified cells able to exercise a regenerative effect and cells of the crystalline lens.

The cells incorporated into the ring will be growing, proliferating and secreting components of the extracellular matrix forming interfibrillar links with other collagens and proteoglycans of the receptor tissue (of the patient) enabling 1) attachment of the annular part to the host tissue and an improvement in the implant's acceptability, particularly important as one of the most common causes of failure in currently used implants in the state of the art is extrusion of the implant by failure of implant-tissue adhesion and 2) a repairing effect occasioned by the live cells.

In a preferred embodiment of the present invention, the annular part for anchoring additionally comprises a therapeutic controlled release system that comprises one or more active substances of ophthalmic interest directed for a particular purpose (e.g., anti-inflammatory, antibiotic, antiviral, anti-tumour, etc.). In a particular embodiment of the invention, this system can be micro and/or nano spheres, capsules or biodegradable micelles that encapsulate the active substances by means of various techniques.

As regards the materials comprising the lens, the material of the central part is preferably of synthetic origin and non-biodegradable, fulfilling the optical requirements of a lens and being capable of being designed to correct various refractive errors (myopia, hyperopia, presbyopia). By the other hand, the material of the annular part acts as the element of union of the lens to the tissue and is a three dimensional matrix able to maintain cells and other components in its interior and preferably is of synthetic origin or mixed and biodegradable or not biodegradable.

In a particular embodiment of the invention, the material, both the central part and the annular part, is a polymeric material. So, in a preferred embodiment, the components included in the annular anchoring part are embedded in said polymeric material.

In another particular embodiment, the polymeric material is a hydrogel. The polymerisation mechanism of these hydrogels can be physical or chemical, ionic or covalent, directed by temperature changes, ionic changes, addition of linking substances or by exposure to radiation.

Some types of hydrogels can be polymerised both *in vivo* and *in vitro* in the presence of photoinitiators using visible or ultraviolet light, converting a monomeric liquid into a gel. These types of hydrogel have advantages over other conventional polymerisation techniques: control over polymerisation area and time, short curing times (from less than one second to a few minutes) at ambient or physiological temperatures and with a minimal production of heat. Another great advantage of photopolymerisation is that hydrogels can be created *in situ* from their aqueous precursors in a minimally invasive way, e.g. using laparoscopy equipment, catheters or subcutaneous injections with transdermic illumination.

Further, photopolymerisable hydrogels have the advantage that they enable the technique of photoencapsulation. This technique has also the great virtue that it enables the incorporation of cells into the initial phase of the gelification process, i.e., it allows mixing of cells and polymers in the liquid phase so that when the gelification of the material is started, the cells are already incorporated into its interior. This is a great advantage for tissue engineering as it solves one of the limitations of this technique, that is the low adhesion effectiveness of cells to the materials in the initial phase of inoculation. This effectiveness diminishes when the support material is porous matrix type as the cells, by simple weight and gravity, fall without any option other than to adhere to the material.

Thus, in a particular embodiment of the invention, the physical method used for hydrogel polymerisation is photopolymerisation, allowing the technique of photoencapsulation to create a hybrid system that forms part of the ring around the lens bio-component and in which the cells and micro/nano particles responsible for release are formed prior to their photoencapsulation in the hydrogel.

The photopolymerisation ability of hydrogels also allows processing of the material and manufacture of the implantable optical system simultaneously.

In a preferred embodiment of the invention, the annular part and the central part comprise at least one hydrogel macromer in common, preferably an acrylic derivative of polyethylene glycol, such as polyethylene glycol diacrylate (PEGDA), to facilitate the subsequent sealing of both parts of the lens. In a particular embodiment, the hydrogel macromer can be copolymerising other monomers/polymers differentially in each part.

The common polymers are soluble in water and present polymerisable zones, preferably by means of a photoinitiator that is activated under UV or visible radiation and that polymerises by means of radical polymerisation. Possible polymerisable regions are acrylates, diacrylates, oligoacrylates, methacrylates, dimethacrylates, oligomethacrylates or any other compound of biological origin susceptible to photopolymerisation.

Some examples are the unsaturated polymers derived from synthetic polymers, such as poly(ethylene oxide), poly(ethylene glycol), poly(vinyl alcohol), poly(vinyl pyrrolidone), poly(amino acids) or derived from natural compounds, such as alginate, hyaluronic acid, chondroitin sulphate, keratan sulphate and quitosane. Also possible are copolymerisation of both types of compound, such as the case of the copolymer of poly(ethylene glycol) and hyaluronic acid.

The annular component can also be constituted by biodegradable polymers, said polymers presenting biodegradable regions and preferably hydrolysable regions, such as ester, peptide, anhydride, orthoester or phosphoester links. Possible polymers susceptible to hydrolysis are the aliphatic polyesters, such as polyglycolic acid (PGA) and its copolymers, polylactic acid (PLA) and its copolymers, or polycaprolactone (PCL) and its copolymers, polyhydroxybutyrates (PHB), polyphosphazenes, polyorthoesters and polycyanoacrylates.

As regards the component materials of the controlled release therapeutic system, these can be synthetic, semi-synthetic and/or natural in origin. The most frequently used among those of synthetic origin are those derived from poly(acrylic acid) and the derivatives of polyesters, mainly poly(epsilon-caprolactone), poly(lactic acid) and the copolymers of lactic acid and glycolic acid. Other materials of semi-synthetic origin can also be used, such as those derived from celluloses with different degrees of solubility, insoluble polymers such as ethyl cellulose and cellulose acetobutyrate and where the solubility depends on the pH, such as cellulose acetophthalate. Natural origin polymers should also be considered, those mainly of a protein nature, such as gelatin and albumin and of polysaccharide nature, such as the alginates, dextran, gum arabic and quitosane.

In a preferred invention, the present invention uses polyesters for manufacture controlled release systems. Polyesters, such as poly(D-lactic-co-glycolic acid) and its derivatives are particularly attractive for polymeric controlled release systems due to their availability, biodegradability, lack of toxicity, bio-compatibility and by being easily combined with a wide variety of active principles.

The encapsulation techniques that are used so that these materials retain active agents are mainly coacervation, interfacial polymerisation, emulsification with evaporation of the solvent and atomisation. In a preferred embodiment, the technique of emulsification and evaporation of the solvent is used.

As regards the active substances that can be incorporated into a controlled release system, these are mainly the anti-inflammatory, antibiotic, anti-viral, anti-tumour, etc., pharmaceuticals with application in ophthalmology. Others of more specific interest may include growth factors, growth factor inhibitors, other cytokines and inhibitors, etc. responsible for corneal function.

In this way, the therapeutic system directs the release of the active compound to benefit tissue repair in the implantation zone. At the same time, being formed from a biodegradable material, the controlled release system will decompose leaving holes in the base material of the ring that will be invaded by cells and adhesive fibres of the matrix, potentiating the adhesive effect of said ring and encouraging the integration of the implant in the stromal bed.

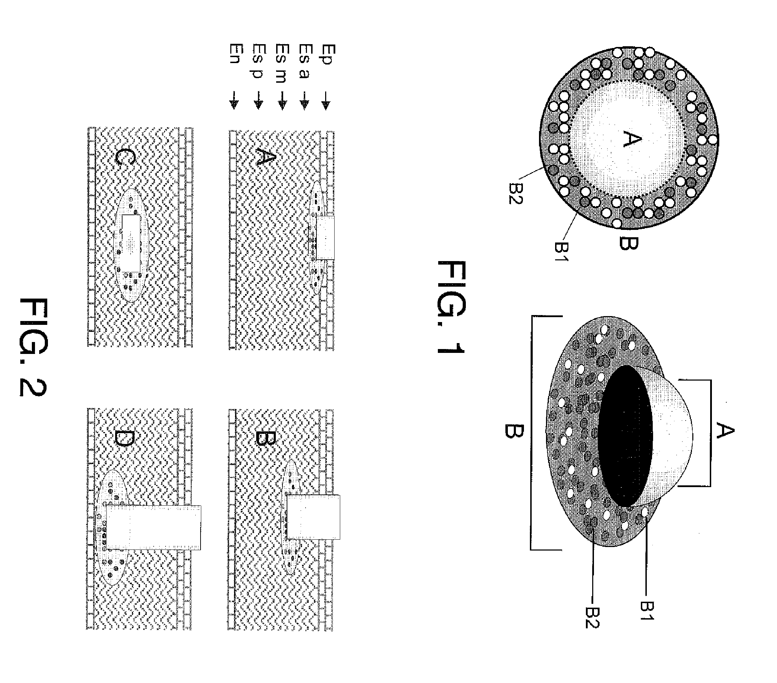

Based on the characteristics described, the composition of the annular or ring part can have three variants:
- Type 1 Ring: comprising cells encapsulated in the polymeric solution;
- Type 2 Ring: comprising active spheres encapsulated in the polymeric solution; and
- Type 3 Ring: comprising cells and active spheres encapsulated in the polymeric solution (Fig. 1).

The concentration of cells and spheres embedded in the annular part can vary depending on system design, spheres size, load/release of the active substance, etc. In preferred embodiments of the invention, in order to retain a good permeability to the passage of nutrients in the ring, the total solid mass of all its components should be between 10% and 30% of the total volume of the formulation. Thus, compositions preserving a constant aqueous content of 70-90%, preferably 80%, are obtained.

Another main aspect of the invention is the procedure for the manufacture of the implantable optical system of the invention, which comprises the following stages:
a. preparation of a solution A comprising a polymeric solution for the formation of the central optical part;
b. preparation of a solution B comprising a polymeric solution and animal cells for the formation of the annular anchoring part;
c. placing solutions A and B independently into specific moulds which include a separator;
d. polymerisation of the system obtained in c) for obtaining individual pseudogels corresponding to the central and annular parts respectively; and
e. removal of the separator and the sealing of the central part with the annular part by polymerisation in order to obtain a bicompartmental lens.

The preparations from stages a) and b) are placed in specific and exclusive moulds for each optical system. The design of the optical part depends mainly on the refractive correction required and the location of lens implantation, which also determines the design of the annular part (Fig. 2). Each lens, therefore, will require a specific thickness and diameter of the optical and annular parts. In preferred embodiments of the invention, the lens has a thickness of the optical part of between 100µm and 600µm, a thickness of the annular part of between 100µm and 300µm and a total diameter of between 6mm and 10mm, of which 4-6mm correspond to the optical part and 2-4mm to the annular part.

In a preferred embodiment, solution B additionally comprises a therapeutic system able to release one or more active substances in a controlled way.

In another particular embodiment, the polymeric solutions A and B additionally comprise photoinitiators, which are activated in stages d) and e) by UV or visible light. They also present short decomposition times, are at least partially soluble in water and have good biocompatibility with cellular compounds.

The photoinitiators used in photopolymerisation are preferably α-hydroxyacetones, phenylglyoxylates, benzyl dimethyl ketals, alfa amino ketones, camphorquinones, such as 2-hydroxy-1- [4-(hydroxyethoxy) phenyl] -2-methyl-1-propanone, or 2,2- dimethoxy-2-phenylacetophenone.

The initiation of polymerisation is accompanied by radiation of UV light with a wavelength in the range of 320-900nm, preferably between 350-370nm.

In such cases, the material from which the mould is made, as well as its design, should allow the passage of UV light so that photopolymerisation can take place in appropriate and constant conditions. The preferred materials are thermoplastic or thermo stable polymeric materials, such as polymethyl methacrylate of methyl polycarbonate, polyvinyl chloride and inorganic glasses, such as quartz with low or zero absorption of UV light.

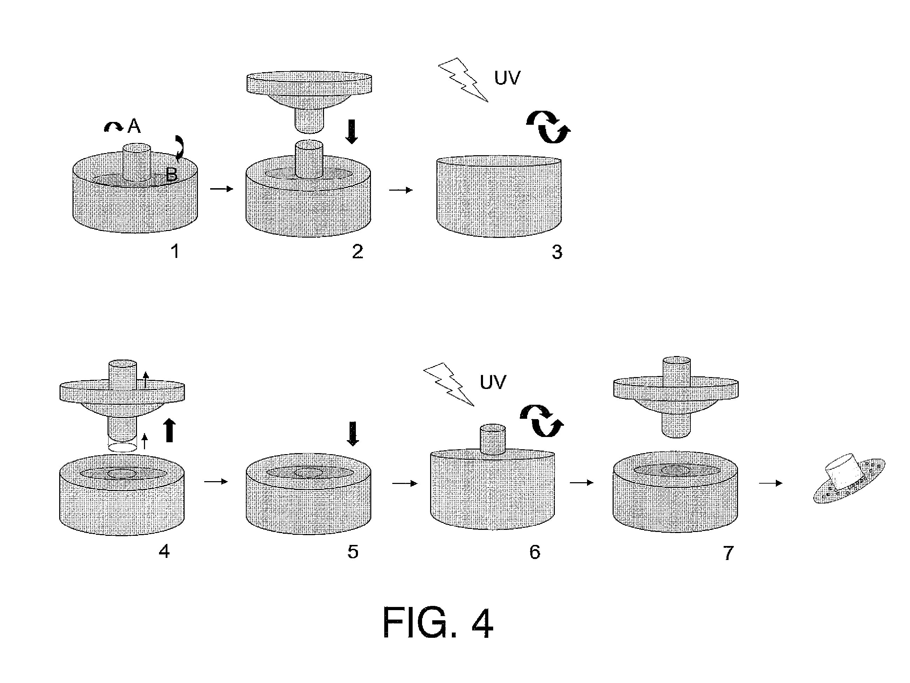

Polymerisation is carried out in two phases: a first phase where two pseudogels are obtained, corresponding to the optical part and the annular part separately, and a second phase where, with the removal of the separator, both optical and annular pseudogels come into contact, allowing cross-linking and union of both parts of the lens (Fig. 4).
The moulds used may have:
- a mobile separator that can be manual or automatic and that can be or not incorporated in the lid of the mould that allows photopolymerisation.
- a coupling system that enables movement of the mould with the aim of ensuring simultaneous photopolymerisation and homogenisation of the samples.

Another main aspect of the invention is an implantable optical system obtained by the procedure described.

The implantable system of the present invention enables the personalisation of the lens depending on the individual requirements of each patient. This can be adapted by the election of different compositions (material, cells and active substances) as well as by the election of different designs with respect to shape, curvature, etc.

Thus, a main aspect of the invention is the use of the described implantable optical system in corneal disorders, specifically in the development of corneal implants, such as intra-stromal lens, for the correction of refractive defects in patients with healthy corneas; as a keratoprosthesis for implantation in the anterior stromal part for patients with defects in more superficial areas (epithelium and anterior stromal part) and as a keratoprosthesis for implantation in the posterior stromal part after removal of the epithelium and stroma in patients with complete stromal defects (Fig. 2).

The implantable optical system described may also be used in other types of ocular disorders, such as disorders of the lens, applied to the development of intra-ocular lenses.

The invention here described is susceptible to variations and modifications not specifically described in this patent application. However, the invention includes all these possible variations and modifications that derive from the state of the art and are therefore obvious to an expert in the field.

We present a non-limiting example embodiment of the present invention below:

### Example 1

### Procedure for obtaining a bicompartmental lens by photopolymerisation (Fig. 3)

### STAGE 1. Preparation of the central optical part (SOLUTION A)

As the base material for the optical and annular parts, poly(ethylene glycol) diacrylate (PEGDA) was used with an average molecular weight of 3400 in a weight/volume proportion of 10% in PBS. 2-hydroxy-1-[4-(hydroxyethoxy) phenyl] -2-methyl-1-propanone was used as the initiator in a proportion of 0.05% weight/volume of the polymeric solution. The initiator solution in 70% ethanol was prepared just before use and was kept on ice and avoiding light incidence.

### STAGE 2. Preparation of the annular or ring part Type 3 (SOLUTION B)

### Obtaining the polymeric solution

The procedure for obtaining the base polymeric solution of the annular part was similar to that described for Phase 1. However, given that in this case the polymeric solution is to receive cells, and to limit the possible cytotoxic effects, the initiator was added at the moment of the incorporation of the mixture into the mould for its subsequent exposure to UV light.

### Obtaining the controlled release therapeutic system

Biodegradable spheres loaded with an active substance were used as the controlled release therapeutic system. In this case, poly(lactic-co-glycolic acid) (PLGA) micro and/or nano spheres were to be used, loaded with dexamethasone (steroid anti-inflammatory widely used in ophthalmology) and a micro/nanoencapsulation technique was employed based on evaporation of solvent from a oil phase/water phase (O/W) emulsion, which involves the dispersion of an organic solution of polymer and the pharmaceutical in a continuous aqueous phase.

To do this, a determined quantity of dexamethasone was added to the polymer dissolved in dichloromethane (internal organic phase). The mixture was homogenised by ultrasound for one minute and added over a determined volume of water and emulsification agent (external aqueous phase). The organic/aqueous (O/W) emulsion was obtained by homogenising in a Polytron homogeniser at 5000rpm for 2 minutes. Then the volume was made up by adding more aqueous phase and homogenising again at the same speed for 1 minute. This mixture was then incorporated into an aqueous solution of greater volume (water and emulsifier) for 3-4 hours with stirring and at ambient temperature until the solvent was completely evaporated. The mature spheres were washed several times in distilled water. Granulometric fractions were separated by sieving. Lastly, they were freeze-dyed at 2-4ºC in a vacuum for storage. Two different embodiments of this type of controlled release system are described below.
- PLGA microspheres loaded with dexamethasone
   The organic phase, comprising 800mg PLGA 50:50 (inherent viscosity 0.17-0.24dL/g), 2ml dichloromethane and 80-160mg dexamethasone, was stirred for 1 minute and exposed to ultrasound on ice for another minute. This solution was added drop by drop on an aqueous phase comprising 5ml polyvinyl alcohol (PVA) at 1% (Pm 72000) and was homogenised for 2 minutes at 5000rpm. The volume was made up to 15ml with polyvinyl alcohol at 0.1 % and was homogenised for 1 more minute. This emulsion was added to a volume of 20ml PVA at 0.1 % and was stirred magnetically for 3-4 hours. The mature microspheres were washed 3 times with distilled water, they were sieved and those of size between 20-50µm were filtered out. Lastly, there were freeze-dyed at 2-4ºC in a vacuum for storage.
- PLGA nanospheres loaded with dexamethasone
   The organic phase, composed of 800mg PLGA 50:50 (inherent viscosity 0.17-0.24dL/g) in 15ml dichloromethane and 200mg dexamethasone dissolved in 15ml acetone was stirred for 5 minutes. This organic phase was then added drop by drop on an aqueous phase of 200ml comprising 5% polyvinyl alcohol (Pm 72000). The emulsion was formed by exposure to ultrasound at 60W for 10 minutes and keeping it on ice. The evaporation of solvent was carried out by magnetic stirring for 12 hours at ambient temperature. The mature nano spheres were washed 3 times with distilled water and were recovered by centrifugation at 35,000rpm for 1 hour at 4ºC. Lastly, there were freeze-dyed at 2-4ºC in a vacuum for storage. *Obtaining primary stromal cells*

Prior to enucleation of the eyes, the area was washed with iodine solution in saline solution. The corneas were extracted using a scalpel (No. 12) and collected in a DMEM-F12 culture medium with an antibiotic solution comprising 1% penicillin and streptomycin kept on ice. The corneas were quartered and placed into an enzyme medium of collagenase (3.3mg collagenase [SIGMA. Ref. C8176] in 1ml DMEM-F12) for 30-45 minutes at 142rpm and at 37ºC. The contents of this first digestion were filtered and the resulting tissue was again resuspended in fresh collagenase medium to carry out another digestion of 1 hour under the same conditions as before (2nd digestion). After a second filtration (keeping the supernatant), the operation was repeated and the content retained in the filter again resuspended in fresh enzymatic medium for two and a half hours at 142rpm and at 37ºC. After this 3rd digestion, the supernatant was collected. The media from the 2nd and 3rd digestions that held the stromal keratocytes were collected, centrifuged and resuspended in a known quantity of DMEM-F12 to measure cellular viability. At this point, there are two options. 1) to encapsulate the primary keratocytes (as will be described in the next sections) or to incubate them to obtain fibroblasts that can also be encapsulated.

### Obtaining the primary keratocytes-PLGA spheres loaded with dexamethasone-PEGDA hydrogel system

A mixture comprising the polymeric PEGDA solution containing the photoinitiator and the PLGA spheres loaded with dexamthasone was prepared. After maintaining this composition in an agitator for one minute, it was tipped over the cellular tablet previously obtained after centrifugation of a cellular suspension of keratocytes for 10 minutes at 4ºC.

Samples with 80% constant aqueous content and 20% solid matter comprising 10% PEGDA, 5% spheres (with a diameter between 500nm and 100µm) and 5% cells (with a diameter of approximately 20µm) were obtained.

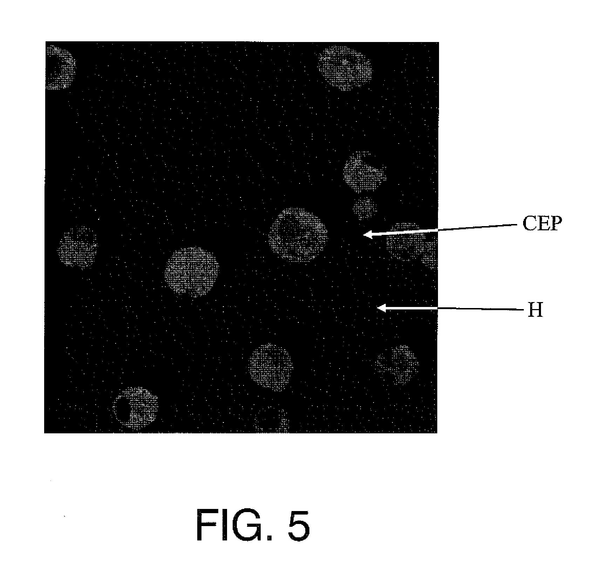

Cellular viability of the encapsulated cells in PEGDA hydrogel was studied using a derivative of fluorescent chloromethyl, which freely diffuses through the membranes of live cells. The red signal at 560nm indicated that the cells were able to remain alive within the hydrogel (Fig. 5).
STAGES 3 and 4. Deposition of the solutions in specific moulds and photopolymerisation of the system to obtain a bicompartmental lens (Fig. 4).

Solutions A and B were placed into the corresponding cavities of the mould (4.1). After closing the mould (4.2), they were subjected to movement for the homogenisation of the samples and they were exposed to UV light to initiate the photopolymerisation (4.3). Thus, in this first stage, two individual pseudogels were obtained under a UV light of intensity 4mW/cm2 and λ= 365nm for 1-2 minutes, corresponding to the optical part and the annular part.

After opening the mould and removing the separator (4.4), the cross-linking and union of both pseudogels (optical and annular) was carried by maintaining the system exposed to UV light of intensity 4mW/cm2 and λ= 365nm for 3-5 minutes more (4.5). Finally after opening the mould, the bicompartmental lens or keratoprosthesis (4.7) was obtained.

The bicompartmental lens obtained had a diameter of 7mm (4mm for the central part and 3mm for the annular part) and a thickness of 0.200mm.

### REFERENCES

1. Whitcher JP, Srinivasan M, Upadhyay MP. Corneal blindness: a global perspective. Bull World Health Organ 2001; 79(3):214-221
2. Aiken-O'Neill, P. and M.J. Mannis, Summary of comeal transplant activity Eye Bank Association of America. Cornea, 2002. 21 (1): p. 1-3.
3. Chirila, T.V., An overview of the development of artificial corneas with porous skirts and the use of PHEMA for such an application. Biomaterials, 2001. 22: p. 3311-3317.
4. Carlsson, D.J., et al., Bioengineered corneas: how close are we? Curr Opin Ophthalmol, 2003. 14(4): p. 192-7.
5. America, E.B.A.O., Eye Banking Statistical Report. 1999, Eye Bank Association of America: Washington, DC.
6. Li, F., et al., Cellular and nerve regeneration within a biosynthetic extracellular matrix for corneal transplantation. Proc Natl Acad Sci U S A, 2003. 100(26): p. 15346-51.
7. Hicks CR, Fitton JH, Chirila TV, Crawford GJ, Constable IJ. Keratoprostheses: advancing toward a true artificial cornea. Surv Ophthalmol 1997; 42(2):175-189.
8. Hicks C, Crawford G, Chirila T, Wiffen S, Vijayasekaran S, Lou X et al. Development and clinical assessment of an artificial cornea. Prog Retin Eye Res 2000; 19(2):149-170.
9. Marchi V, Ricci R, Pecorella I, Ciardi A, Di Tondo U. Osteo-odonto-keratoprosthesis. Description of surgical technique with results in 85 patients. Cornea 1994; 13(2):125-130.
10. Doane MG, Dohlman CH, Bearse G. Fabrication of a keratoprosthesis. Cornea 1996; 15(2):179-184.
11. Teichmann KD, al Hussain HM, Karcioglu ZA. Long-term complications of Strampelli's osteo-odonto-keratoprosthesis. Aust N Z J Ophthalmol 1996; 24(2):158-159.
12. Cardona H. Keratoprosthesis with a plastic fiber meshwork supporting plate. Report of an experimental and comparative histologic study. Am J Ophthalmol 1967; 64(2):228-233.
13. Khan AO. Visual sensation in cataract surgery. Ophthalmology 2001; 108(12):2157-2158.
14. Hicks CR, Lou X, Platten S, Clayton AB, Vijayasekaran S, Fitton HJ et al. Keratoprosthesis results in animals: an update. Aust N Z J Ophthalmol 1997; 25 Suppl 1:S50-S52.
15. Dohlman CH, Terada H. Keratoprosthesis in pemphigoid and Stevens-Johnson syndrome. Adv Exp Med Biol 1998; 438:1021-1025.
16. Nouri M, Terada H, Alfonso EC, Foster CS, Durand ML, Dohlman CH. Endophthalmitis after keratoprosthesis: incidence, bacterial causes, and risk factors. Arch Ophthalmol 2001; 119(4):484-489.
17. Hsiue GH, Lee SD, Chang PC. Platelet adhesion and cellular interaction with poly(ethylene oxide) immobilized onto silicone rubber membrane surfaces. J Biomater Sci Polym Ed 1996; 7(10):839-855.
18. Pintucci S, Perilli R, Formisano G, Caiazza S. Influence of dacron tissue thickness on the performance of the Pintucci biointegrable keratoprosthesis: an in vitro and in vivo study. Cornea 2001; 20(6):647-650.
19. Crawford GJ, Constable IJ, Chirila TV, Vijayasekaran S, Thompson DE. Tissue interaction with hydrogel sponges implanted in the rabbit cornea. Cornea 1993; 12(4):348-357.
20. Hicks CR, Chirila TV, Dalton PD, Clayton AB, Vijayasekaran S, Crawford GJ et al. Keratoprosthesis: preliminary results of an artificial corneal button as a full-thickness implant in the rabbit model. Aust N Z J Ophthalmol 1996; 24(3):297-303.
21. Hicks CR, Vijayasekaran S, Chirila TV, Platten ST, Crawford GJ, Constable IJ. Implantation of PHEMA keratoprostheses after alkali burns in rabbit eyes. Cornea 1998; 17(3):301-308.
22. Hicks CR, Chirila TV, Clayton AB, Fitton JH, Vijayasekaran S, Dalton PD et al. Clinical results of implantation of the Chirila keratoprosthesis in rabbits. Br J Ophthalmol 1998; 82(1):18-25.
23. Sandeman SR, Faragher RG, Allen MC, Liu C, Lloyd AW. Novel materials to enhance keratoprosthesis integration. Br J Ophthalmol 2000; 84(6):640-644.
24. Legeais JM, Renard G, Parel JM, Savoldelli M, Pouliquen Y. Keratoprosthesis with biocolonizable microporous fluorocarbon haptic. Preliminary results in a 24-patient study. Arch Ophthalmol 1995; 113(6):757-763.
25. Renard G, Cetinel B, Legeais JM, Savoldelli M, Durand J, Pouliquen Y. Incorporation of a fluorocarbon polymer implanted at the posterior surface of the rabbit cornea. J Biomed Mater Res 1996; 31 (2):193-1999
26. Renard G. [Artificial cornea]. Bull Acad Natl Med 1996; 180(3):659-665.
27. Caldwell DR. The soft keratoprosthesis. Trans Am Ophthalmol Soc 1997; 95:751-802.
28. Hicks CR, Crawford GJ, Lou X, Tan DT, Snibson GR, Sutton G et al. Corneal replacement using a synthetic hydrogel cornea, AlphaCor: device, preliminary outcomes and complications. Eye 2003; 17(3):385-392.
29. Dohlman, C., A recollection from Boston. Experimental Eye Research, 2004. 78(3): p.xxi-xxii.
30. Hicks, C.R. and G.J. Crawford, Melting after keratoprosthesis implantation: the effects of medroxyprogesterone. Cornea, 2003. 22(6): p. 497-500.
31. Hicks, C.R., et al., Outcomes of implantation of an artificial cornea alphacor: effects of prior ocular herpes simplex infection. Cornea, 2002. 21 (7): p. 685-690.
32. Germain, L., et al., Reconstructed human cornea produced in vitro by tissue engineering. Pathobiology, 1999. 67(3): p. 140-7.
33. Griffith, M., et al., Functional human corneal equivalents constructed from cell lines. Science, 1999. 286(5447): p. 2169-72.
34. Orwin, E.J. and A. Hubel, In vitro culture characteristics of corneal epithelial, endothelial, and keratocyte cells in a native collagen matrix. Tissue Eng, 2000. 6(4): p.307-19.
35. Schneider, A.I., K. Maier-Reif, and T. Graeve, Constructing an in vitro cornea from cultures of the three specific corneal cell types. In Vitro Cell Dev Biol Anim, 1999.35(9): p. 515-26.
36. Griffith, M. et al. "Artificial Cornea". U.S. 6.645.715, November 11th, 2003. Appl. 09/624909, July 24th, 2000. p45.
37. Cukierman, E., et al., Taking cell-matrix adhesions to the third dimension. Science, 2001. 294(5547): p. 1708-12.
38. Scharp, D. et al. Implantation of encapsulated biological materials for treating diseases. US20040136971, July 15th, 2004. Appl. 10/684,859, Oct. 14th, 2003. p. 1-66.
39. Bhatia, S.N. et al. Three dimensional cell patterned biopolymer scaffolds and method of making the same. US20050169962, Aug. 4th, 2005. Appl. 11/035,394, Jan. 12th, 2005. p. 1-25.
40. Chirila, T.V. et al. Keratoprosthesis. US 5300116, April 5th, 2004. Appl. 931027, Aug. 14th, 1992.
41. Chirila, T.V. et al. Method of producing a Keratoprosthesis. US 5458819, Oct. 17th ,1995. Appl. 170379, Dec. 20th, 1993.
42. Caldwell Delmar, R. et al. Intraocular prótesis. US 4865601, Sept 12th, 1989. Appl. 70783, Jul. 7th, 1987.
43. Caldwell Delmar, R. et al. Intraocular prótesis. US 4932968, June 12th, 1990. Appl. 391887, Aug. 9th, 1989.
44. Jacob-Labarre, J. Intraocular prótesis. US 5282851, Feb. 1st, 1994. Appl. 837483, feb. 18th, 1992.
45. Elisseeff J, Mclntosh W, Anseth K, Riley S, Ragan P, Langer R. Photoencapsulation of chondrocytes in poly(ethylene oxide)-based semi-interpenetrating networks. Journal of Biomedical Materials Research 2000;51 (2)
46. Wang D, Williams CG, Yan F, Cher N, Lee H, Elisseeff JH. Bioresponsive phosphoester hydrogels for bone tissue engineering. Tissue Engineering 2005; 11 (1-2):201-213.
47. Williams CG, Kim TK, Taboas A, Malik A, Manson P, Elisseeff J. In Vitro Chondrogenesis of Bone Marrow-Derived Mesenchymal Stem Cells in a Photopolymerizing Hydrogel. Tissue Engineering 2003; 9(4): 679-688
48. Nuttelman CR, Tripodi MC, Anseth KS. Synthetic hydrogel niches that promote hMSC viability. Matrix Biology 2005;24(3):208-218.
49. Miles-Thomas J, Elisseeff JH, Morales N, Frimberger D, Gearhart JP, Lakshmanan Y. Human stem cells in a photopolymerizable hydrogel: The next generation of engineered tissue. Journal of Urology 2004; 171(4 Supplement):46.
50. Alhadlaq A, Elisseeff JH, Hong L, Williams CG, Caplan Al, Sharma B, Kopher RA, Tomkoria S, Lennon DP, Lopez A, Mao, Jeremy J. Adult stem cell driven genesis of human-shaped articular condyle. Annals of Biomedical Engineering 2004;32(7): 911-923.
51. Bryant, Stephanie J.; Nuttelman, Charles R.; Anseth, Kristi S.. Cytocompatibility of UV and visible light photoinitiating systems on cultured NIH/3T3 fibroblasts in vitro. Journal of Biomaterials Science Polymer Edition, (2000) Vol. 11, No. 5
52. Cruise, Gregory M.; Hegre, Orion D.; Scharp, David S.; Hubbell, Jeffrey A.. A sensitivity study of the key parameters in the interfacial photopolymerization of poly(ethylene glycol) diacrylate from porcine islets. Biotechnology and Bioengineering, (1998) Vol. 57, No. 6, pp. 655-665.
53. Masters, Kristyn S.; Shah, Darshita N.; Leinwand, Leslie A.; Anseth, Kristi S. Crosslinked hyaluronan scaffolds as a biologically active carrier for valvular interstitial cells. Biomaterials (2005), 26(15), 2517-2525
54. Quick, Deborah J.; Anseth, Kristi S. DNA delivery from photocrosslinked PEG hydrogels: encapsulation efficiency, release profiles, and DNA quality. Journal of Controlled Release, (April 28 2004) Vol. 96, No. 2, pp. 341-351.
55. Ramakumar, Sanjay [Reprint Author]; Phull, Hardeep; Purves, Todd; Funk, Joel; Copeland, Duan; Ulreich, Judith B.; Lai, Li-Wen; Lien, Yeong-Hau. Howard Novel delivery of oligonucleotides using a topical hydrogel tissue sealant in a murine partial nephrectomy model. Journal of Urology, (SEP 2005) Vol. 174, No. 3, pp. 1133-1136.
56. Elisseeff, Jennifer; Mclntosh, Winnette; Fu, Karen; Blunk, Torsten; Langer, Robert. Controlled-release of IGF-I and TGF-β1 in a photopolymerizing hydrogel for cartilage tissue engineering. Journal of Orthopaedic Research (2001), 19(6), 1098-1104
57. Patton, Jaqunda N.; Palmer, Andre F. Photopolymerization of Bovine Hemoglobin Entrapped Nanoscale Hydrogel Particles within Liposomal Reactors for Use as an Artificial Blood Substitute. Biomacromolecules (2005), 6(1), 414-424
58. Hill-West JL, Chowdhury SM, Slepian MJ, Hubbell JA. Inhibition of thrombosis and intimal thickening by in situ photopolymerization of thin hydrogel barriers. Proc Natl Acad Sci U S A 1994; 91 (13):5967-5971.
59. West JL, Hubbell JA. Separation of the arterial wall from blood contact using hydrogel barriers reduces intimal thickening after balloon injury in the rat: the roles of medial and luminal factors in arterial healing. Proc Natl Acad Sci U S A 1996; 93(23):13188-13193.
60. Quinn CP, Pathak CP, Heller A, Hubbell JA. Photo-crosslinked copolymers of 2-hydroxyethyl methacrylate, poly(ethylene glycol) tetraacrylate and ethylene dimethacrylate for improving biocompatibility of biosensors. Biomaterials 1995; 16(5):389-396.
61. Edman P. Solid microspheres as drug delivery systems. En: Sartoreli AC, ed. Methods of drug delivery. New York:Pergamon Press;1985:23.
62. Julienne MC, Alonso Ma J, Gómez JL, Benoit JP. Preparation of poly (DL lactide/glycolide) nanoparticles of controlled particle size distribution: application of experimental designs. Drug Dev Ind Pharm 1992;(18):1063.
63. Shen Wu X. Preparation, characterization, and drug delivery applications of microspheres based on biodegradable lactic/glycolic acid polymers. En: Wise LM, Trantolo DJ, Altobelli DE, Yaszemski MJ, Gresser JD, Schwartz, eds. Encyclopedic handbook of biomaterials and bioengineering. New York: Marcel Dekker; 1995:1151-200.
64. Hubbel JA. et al. Gels for encapsulation of biological materials US 5801033, September 1st, 1998; App. US480678, june 7th, 1995. p 32.
65. Hubbel JA, et al. Photopolymerizable biodegradable hydrogels as tissue contaction materials as controlled-release carriers. US2002091229, July 11th, 2002. App. 10/021508, October 22, 2001.
66. Lee DA. Drug delivery device (US19894863457).
67. Ohtori A, et al. Controlled-release pharmaceutical preparation for intra-ocular implant. EP 0488401, June 3rd, 1992. App. EP91120586.2, November 11th, 1989.
68. Wong VG. Biodegradable ocular implants. US 5164188, November 17th, 1992. App. 440344, November 22, 1989.
69. Huang GT, et al. Sustained release intraocular implants and related methods. US2005244468, November 3rd, 2005. App. US10837356, April 30th, 2004.
70. Hughes PM, Malone et al. Macromolecule-containing sustained release intraocular implants and related methods. US2005281861, December 22, 2005. App. 1116698, april 27, 2005.
71. Jimenez AM, et al. Implants and microspheres for the sustained release of drugs for ophthalmic use and preparation methods thereof. W02006/028361, march 16, 2006.
72. Marmo JC and Back A. Device and Methods for Improving Vision US 2005/0080484, April 14, 2005. App. 10661400, September 12, 2003.

## Claims

1. Implantable optical system comprising a central optical part and an annular part for anchoring, **characterised in that** this annular part comprises animal cells, including human cells.

2. Implantable optical system according to claim 1, **characterised in that** the cells come from the patient who will receive the implant and/or from a donor.

3. Implantable optical system according to claims 1 or 2, **characterised in that** the cells are selected from stem cells, primary keratocytes, fibroblasts, myofibroblasts, genetically modified cells capable of exercising a regenerative effect and cells of the crystalline lens.

4. Implantable optical system according to any of the previous claims, **characterised in that** the annular part for anchoring additionally comprises a controlled release therapeutic system that comprises one or more active substances.

5. Implantable optical system according to claim 4, **characterised in that** the therapeutic system is micro and/or nano spheres, capsules or biodegradable micelles type.

6. Implantable optical system according to any of the previous claims, **characterised in that** the material of the optical part is of synthetic origin and non biodegradable.

7. Implantable optical system according to any of the previous claims, **characterised in that** the material of the annular part is of synthetic, natural or mixed origin and biodegradable or non biodegradable.

8. Implantable optical system according to claims 6 and 7, **characterised in that** the material of the central and annular parts is a polymeric material.

9. Implantable optical system according to claim 8, **characterised in that** the components comprised in the annular anchoring part are embedded in the polymeric material.

10. Implantable optical system according to claim 9, **characterised in that** the polymeric material of both the central part and the annular part is a hydrogel type polymeric material.

11. Implantable optical system according to claim 10**, characterised in that** the annular and central parts comprise at least one hydrogel type macromer in common.

12. Implantable optical system according to claim 11, **characterised in that** the macromer is copolymerising other monomers and/or polymers differentially in each part.

13. Implantable optical system according to claim 12, **characterised in that** said macromer is an acrylic derivative of polyethylene glycol.

14. Implantable optical system according to claim 13, **characterised in that** said macromer is a polyethylene glycol diacrylate (PEGDA).

15. Implantable optical system according to any of the claims 10-14, **characterised in that** the hydrogel polymerises by physical or chemical methods.

16. Implantable optical system according to claim 15, **characterised in that** the physical method used is photopolymerisation.

17. Implantable optical system according to any of the previous claims, **characterised in that** the composition of the annular part has an aqueous percentage content that varies between 70% and 90%.

18. Procedure for the development of an implantable optical system according to claims 1-17, **characterised in that** it comprises the following stages:
a. preparation of a solution A comprising a polymeric solution for the formation of the central optical part;
b. preparation of a solution B comprising a polymeric solution and animal cells for the formation of the annular part for anchoring;
c. placing solutions A and B independently into specific moulds that have a separator;
d. polymerisation of the system obtained in c) for obtaining individual pseudogels corresponding to the central and annular parts respectively; and
e. removal of the separator and the sealing of the central part with the annular part by polymerisation in order to obtain a bicompartmental lens.

19. Procedure according to claim 18 **characterised in that** solution B additionally comprises a therapeutic system able to release one or more active substances in a controlled way.

20. Procedure according to claim 18, **characterised in that** solutions A and B additionally comprise a photoinitiator.

21. Procedure according to claim 20, **characterised in that** stages d) and e) are carried out with UV or visible light.

22. Procedure according to claim 21, **characterised in that** the UV light has a wavelength in the range 320-900nm.

23. Procedure according to claim 22, **characterised in that** the UV light has a wavelength in the range 350-370nm.

24. Implantable optical system obtained by the procedure of claims 18-23.

25. Use of an implantable optical system according to claims 1-17 and 24 in corneal disorders.

26. Use of an implantable optical system according to claim 25 in the development of corneal implants.

27. Use of an implantable optical system according to claim 26, in the development of an intra-stromal lens or a keratoprosthesis.

28. Use of an implantable optical system according to claim 1 in disorders of the crystalline lens.

29. Use of an implantable optical system according to claim 28, in the development of an intro-ocular lens.
